# EUROPEAN PATENT SPECIFICATION

(11) **EP 3 656 299 B1**
(45) Date of publication and mention of the grant of the patent: **25.11.2026**
(21) Application number: 19183973.7
(22) Date of filing: 02.07.2019
(51) Int. Cl.: A61B 5/00, G16H 50/30, G16H 50/70, A61B 5/372, A61B 5/384

(54) **SYTEM FOR NEUROLOGICAL EVENT DETECTION**
SYSTEM ZUR ERKENNUNG NEUROLOGISCHER EREIGNISSE
SYSTÈME DE DÉTECTION D'ÉVÉNEMENT NEUROLOGIQUE

(30) Priority: 21.11.2018 TW 107141428
(43) Date of publication of application: 27.05.2020
(73) Proprietor: A-Neuron Electronic Corporation, Zhubei City, Hsinchu 30273 (TW)
(72) Inventor: CHANG, Chia-Chi, Taipei City 116 (TW); LIN, Pei-Chen, Hsinchu City 300 (TW); HSIN, Yue-Loong, Taichung City 408 (TW)
(74) Representative: Emde, Eric

(56) References cited:
- US-A1- 2002 103 512
- US-A1- 2012 172 743
- DUO CHEN ET AL: "A high-performance seizure detection algorithm based on Discrete Wavelet Transform (DWT) and EEG", PLOS ONE, vol. 12, no. 3, 9 March 2017 (2017-03-09), pages e0173138, XP055646914, DOI: 10.1371/journal.pone.0173138

## Description

### BACKGROUND

### Field of the Invention

The invention relates to a method and a system for neurological event detection.

### Description of Related Art

Epilepsy is a common syndrome among chronic neurological diseases, and about 60 million people in the world suffer from epilepsy, while approximately 30% among them are still unable to effectively control the seizure by currently available antiepileptic drugs, and need non-pharmacological adjuvant therapies. Even though an epilepsy surgery may help, there are some patients who are not suitable for treatments of brain resection and are only allowed to select neuromodulation for mitigating severe seizures. Either the conventional epilepsy surgery or the new neuromodulation needs accurate analysis and determination for onset brainwaves to locate a seizure onset zone in the brain or determine an onset time by an implant of the neuromodulation.

A conventional diagnosis using electroencephalography (EEG) focuses on the detection of symptoms and pre-stage characteristics of the seizure, but clinically demands further require identifying and labeling a whole segment of the epilepsy. However, such demand cannot be satisfied by any current technique because of nothing but the difficulties in interpretation caused by discontinuity of the brainwave characteristics.

Documents US2002/103512A1 and US2012/172743A1 both disclose detection of events in EEGs using classification based on sliding windows.

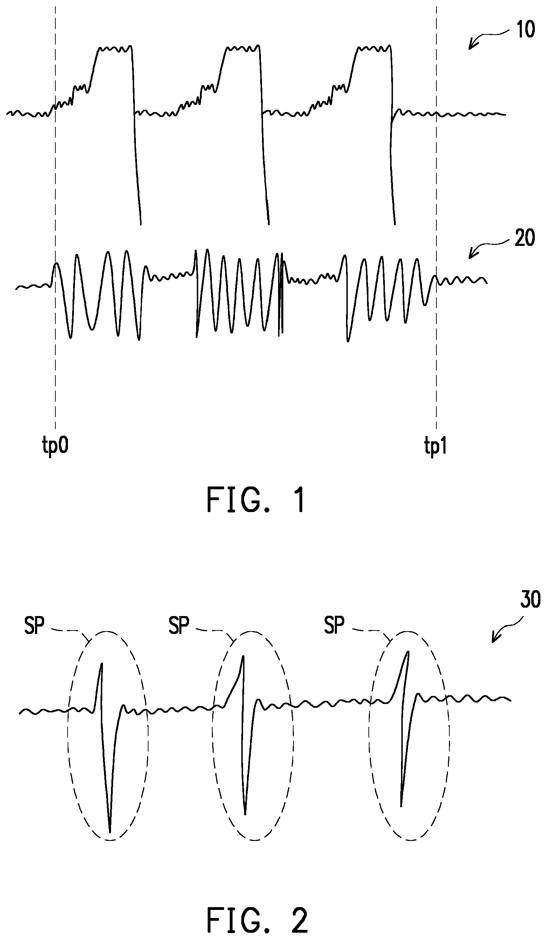

Referring to FIG. 1, discrete brainwaves are characterized in, for example, intermittent onset brainwaves 10 and 20, which may also be referred to as burst suppression, and commonly found in a part of later seizure onset of the brainwaves. In terms of the intermittent onset brainwaves 10 and 20, even though time periods tp0 to tp1 belong to the same seizure state, it is usually erroneously determined as multiple seizure states with shorter durations in past diagnoses that the time periods tp0 to tp1 of the entire seizure occurrence cannot be correctly identified and labeled.

In addition, referring to FIG. 2, brainwaves 30 without seizures may have multiple spikes SP (spike) due to a transient abnormal discharge, and the misjudgment of the seizure usually occurs due to these spikes SP in past diagnoses.

### SUMMARY

The invention is defined by the appended claims. None of the methods described, hereby, forms parts of the claimed invention. Accordingly, the embodiments of the invention provide a method and a system for neurological event detection capable of enhancing the accuracy of detecting the occurrence of a neurological event.

According to an embodiment of the disclosure, a method for neurological event detection is provided, which includes the following steps. A neural oscillation signal is obtained, and a plurality of features are extracted from the neural oscillation signal. A plurality of classification results corresponding to a plurality of times are obtained according to the plurality of features by using a classification model, wherein the classification model is constructed based on a plurality of training data on whether the neurological event occurs is known. A final determination result corresponding to each of a plurality of evaluation time windows is calculated according to a preset time window width and the plurality of classification results, and the final determination results are displayed. Each of the final determination results indicates whether the neurological event occurs, and each of the evaluation time windows corresponds to the plurality of classification results and one final determination result.

According to an embodiment of the invention, a system for neurological event detection system including a detector, a processor and a display is provided. The detector is configured to obtain a neural oscillation signal. The processor is coupled to the detector and is configured to extract a plurality of features from the neural oscillation signal, obtain a plurality of classification results corresponding to a plurality of times according to the plurality of features by using a classification model, wherein the classification model is constructed based on a plurality of training data on whether a neurological event occurs is known, and calculate a final determination result corresponding to each of a plurality of evaluation time windows according to a preset time window width and the plurality of classification results. The display is coupled to the processor and is configured to display the final determination results. Each of the final determination results indicates whether the neurological event occurs, wherein each of the evaluation time windows corresponds to the plurality of classification results and one final determination result.

In order to make the aforementioned and other features and advantages of the invention more comprehensible, several embodiments accompanied with figures are described in detail below.

### BRIEF DESCRIPTION OF THE DRAWINGS

The accompanying drawings are included to provide a further understanding of the invention, and are incorporated in and constitute a part of this specification. The drawings illustrate embodiments of the invention and, together with the description, serve to explain the principles of the invention.
FIG. 1 is a schematic diagram illustrating intermittent onset brainwaves of the related art.
FIG. 2 is a schematic diagram illustrating a transient abnormal discharge of a brainwave of the related art.
FIG. 3 is a block diagram illustrating a system for neurological event detection according to an embodiment of the invention.
FIG. 4 is a flowchart illustrating a method for neurological event detection according to an embodiment of the invention.
FIG. 5 is a schematic diagram illustrating a plurality of classification results corresponding to a plurality of times according to an embodiment of the invention.
FIG. 6 is a schematic diagram illustrating the evaluation time windows according to an embodiment of the invention.
FIG. 7 is a schematic diagram illustrating displaying of final determination results according to an embodiment of the invention.
FIG. 8 is a schematic diagram illustrating a plurality of classification results corresponding to a plurality of times according to another embodiment of the invention.
FIG. 9 is a schematic diagram illustrating the evaluation time windows according to another embodiment of the invention.
FIG. 10 is a schematic diagram illustrating displaying of final determination results according to another embodiment of the invention.

### DESCRIPTION OF EMBODIMENTS

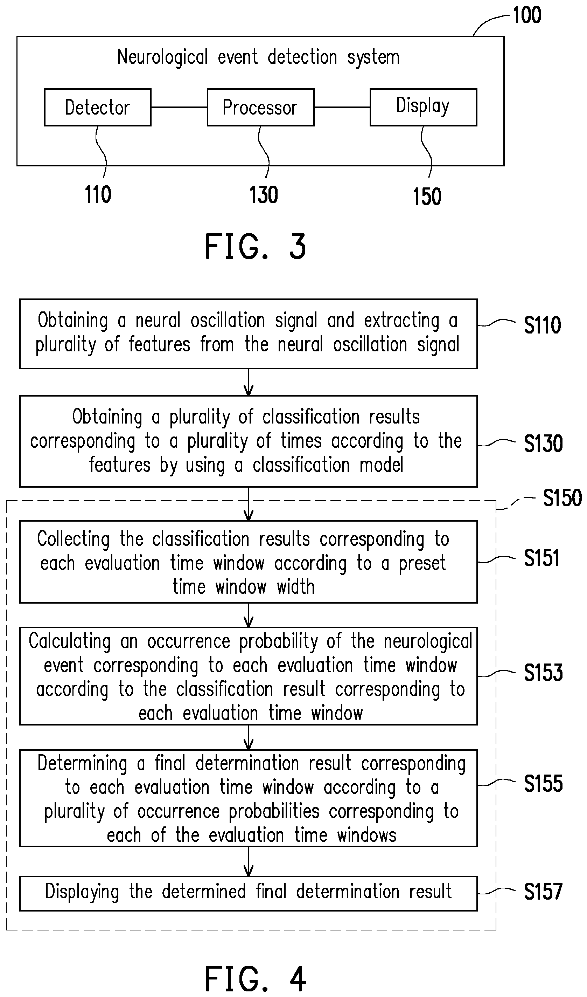

FIG. 3 is a block diagram illustrating a system for neurological event detection according to an embodiment of the invention.

Referring to FIG. 3, a neurological event detection system 100 includes a detector 110, a processor 130 and a display 150, wherein the detector 110 and the display 150 are coupled to the processor 130. The neurological event detection system 100 is adapted to detect whether a neurological event occurs, and a determination result is displayed through the display 150. As such, the time for artificially reviewing brainwaves and determining whether the neurological event occurs may be dramatically reduced. In the embodiment, a detected neurological event is, for example, epilepsy, but the invention is not limited thereto.

The detector 110 is configured to obtain a neural oscillation signal of a subject. In the embodiment, the detector 110 includes electrodes of multiple (for example, 64) channels, which are respectively disposed at locations corresponding to different brainwave zones of the subject, thereby obtaining electroencephalography (EEG) signals of the subject. In other embodiments, the detector 110 may also obtain other neural oscillation signals in other manners, which is not limited in the invention. It should be mentioned that the multiple electrodes of the multiple channels may obtain a plurality of neural oscillation signals in parallel, and for the sake of conciseness, one of the neural oscillation signals is illustrated as an example for description in the specification. The specification may be applied to the neural oscillation signal obtained by each electrode.

The processor 130 is configured to analyze a neurological event corresponding to a neural oscillation signal obtained by the detector 110 to determine an occurrence time of the neurological event. In the embodiment, the neurological event analyzed by the processor 130 is epilepsy, and the processor 130 identifies an occurrence time of the epilepsy according to the neural oscillation signal. A specific analysis method will be described in detail below. The processor 130 may be, for example, a dual-core, quad-core, or octa-core central processing unit (CPU), a system-on-chip (SOC), an application processor, a media processor, a microprocessor, a digital signal processor, a programmable controller, application specific integrated circuits (ASIC), a programmable logic device (PLD) or other similar devices or a combination of these devices, but the invention is not limited thereto.

The display 150 is configured to display a determination result with respect to the neurological event by the processor 130 for a doctor or a user to accurately locate a time that the neurological event may occur according to the content displayed by the display 150. In the embodiment, the display 150 not only displays electroencephalography (EGG) signals but also shows an EGG signal during a seizure period in a different color. In other embodiments, the display 150 may also display analysis and determination results of the processor 130 in other different manners, and the invention is not limited thereto.

Hereinafter, a description related to a neurological detection method of the embodiments of the invention will be set forth below.

FIG. 4 is a flowchart illustrating a method for neurological event detection according to an embodiment of the invention.

The method for neurological event detection introduced in the embodiment illustrated in FIG. 4 is adapted to the neurological event detection system 100 of the embodiment illustrated in FIG. 3, and thus, the description will be made with reference to each element of the neurological event detection system 100.

First, the processor 130 obtains a neural oscillation signal of a subject through the detector 110, and then extracts a plurality of features of the neural oscillation signal (step S110). Specifically, an oscillation signal includes temporal information and spatial information, wherein the temporal information includes a frequency domain feature, a time domain feature, a feature for measuring information theory and so on. For example, the frequency domain feature includes a total power, a peak frequency of spectrum, a spectral edge frequency and so on. The time domain feature includes a line length, a number of maxima, a number of minima, a root mean squared amplitude and so on. The measurement of the information theory includes a Shannon entropy, a singular value decomposition entropy, Fisher information and so on. Details related to the features of the oscillation signal may be obtained by the persons with ordinary skills in the art from related literatures and thus, will not be repeated.

In the embodiment, the processor 130 obtains an EGG signal of the subject and then extracts a plurality of features of the EGG signal, such as a spectrum feature ranging from 1 to 61 Hz, a complexity index (e.g., an approximate entropy) and a waveform index (e.g., a line length), but the invention is not limited thereto.

Then, the processor 130 uses a classification model to obtain a plurality of classification results corresponding to a plurality of times according to the extracted features (step S130), wherein the classification results are used to indicate whether a neurological event occurs. Specifically, the classification model is constructed based on a plurality of training data in which whether a neurological event occurs is labeled. The classification model includes, for example, an artificial neural network (ANN) model, a support vector machine (SVM) and linear classification model, a fuzzy logic model and an auto-learn system etc.

In the embodiment, the classification model uses a linear classification model. The processor 130 obtains in advance a plurality of EGG signals with known seizure occurrences to obtain a plurality of features of the EGG signals as the training data. Then, the processor 130, for example, forms a plurality of feature matrices with the plurality of features, and labels messages regarding whether epilepsy seizure occurs in the plurality of feature matrices. Accordingly, the feature matrices labeled with whether the epilepsy seizure occurs are used to train the linear classification model (for example, by fine-tuning parameters and weights of the model and so on), such that the trained linear classification model is capable of classifying a plurality of input features into one of classes indicating the occurrence of seizure and the non-occurrence of seizure.

In the embodiment, according to the features extracted in step S110, the processor 130 uses the trained linear classification model to predict whether the seizure occurs in each time period of the EGG signal. For example, if the processor 130 analyzes and calculates the features of the EGG signal according to a sliding window having a time width of 1 second (sec), the sliding windows overlap by 80% with each other, and each time period is 0.2 sec. However, the invention is not intent to limit the length of each time period.

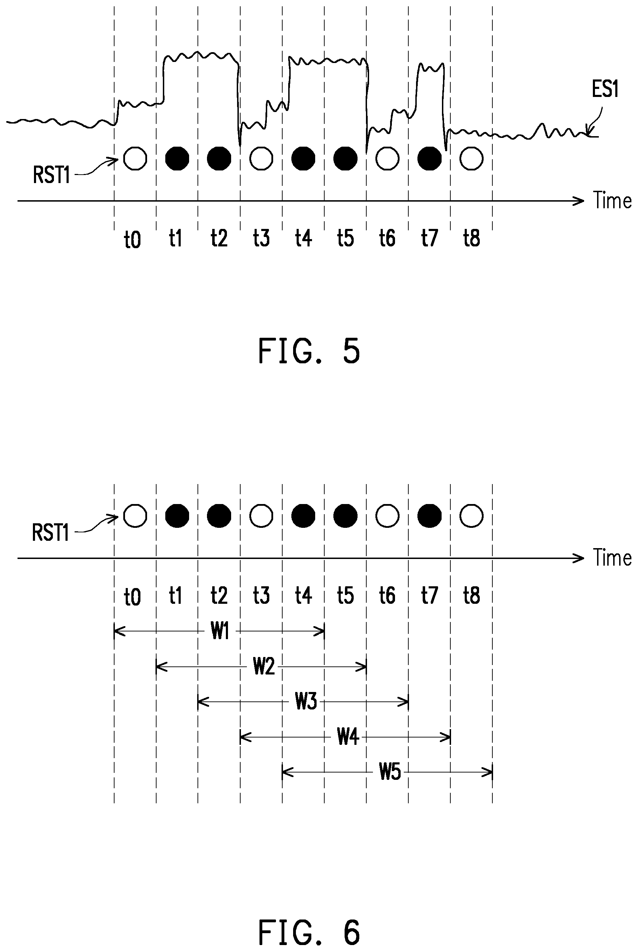

FIG. 5 is a schematic diagram illustrating a plurality of classification results corresponding to a plurality of times according to an embodiment of the invention.

Referring to FIG. 5, each of time periods t0 to t8 is, for example, 0.2 sec, a classification result RST1 with the occurrence of seizure is represented by a black circle, and a classification result RST1 without the occurrence of seizure is represented by a white circle. Taking an intermittent onset EGG signal ES1 as an example, the processor 130 extracts a plurality of features of the EGG signal ES1 in the time period t0 and classifies the features by using a classification model. As illustrated in FIG. 5, the classification result RST1 in the time period t0 indicates that the seizure does not occur. In addition, the processor 130 extracts a plurality of features of the EGG signal ES1 in the time period t1 and then classifies the features by using the classification model. As illustrated in FIG. 5, the classification result RST1 in the time period t1 indicates that the seizure occurs. By deriving by analogy in this way, the classification results RST1 of the EGG signal ES1 from the time period t0 to the time period t8 respectively indicate the non-occurrence of seizure, the occurrence of seizure, the occurrence of seizure, the non-occurrence of seizure, the occurrence of seizure, the occurrence of seizure, the non-occurrence of seizure, the occurrence of seizure, and the non-occurrence of seizure.

Then, the processor 130 calculates a final determination result corresponding to each of a plurality of evaluation time windows according to a preset time window width and the plurality of classification results and displays the final determination results by using the display 150 (step S150), wherein the final determination result indicates whether the neurological event occurs. Specifically, the preset time window width is used to determine a time length of each evaluation time window, and an evaluation time window corresponds to a plurality of classification results and a final determination result. To be more specific, after obtaining the plurality of classification results corresponding to the plurality of times by using the classification model, the processor 130 does not directly use the classification results as the final determination results of whether the neurological event occurs, but comprehensively evaluates the plurality of classification results within a period of time, so as to obtain one final determination result.

To be detailed, the processor 130 first collects the plurality of classification results corresponding to each evaluation time window according to the preset time window width (step S151), then calculates an occurrence probability of the neurological event corresponding to each evaluation time window according to the classification results corresponding to each evaluation time window (step S153), determines the final determination result corresponding to each evaluation time window according to the plurality of occurrence probabilities corresponding to the plurality of evaluation time windows (step S155), and finally displays the determined final determination result through the display 150 (step S157).

FIG. 6 is a schematic diagram illustrating the evaluation time windows according to an embodiment of the invention.

Referring to FIG. 6, the processor 130 obtains the classification results RST1 of the EGG signal ES1 from the time period t0 to the time period t8 in step S130. In the embodiment, the preset time window width is, for example, 1 sec, thus, an evaluation time window includes 5 time periods, and each time period has a classification result. For example, an evaluation time window W1 includes the time periods t0 to t4, an evaluation time window W2 includes the time periods t1 to t5, an evaluation time window W3 includes the time periods t2 to t6, an evaluation time window W4 includes the time periods t3 to t7, an evaluation time window W5 includes the time periods t4 to t8, and so on.

It should be mentioned that for descriptive convenience, only the 5 evaluation time windows W1 to W5 are taken as an example, however, the persons with ordinary skills in the art should understand that the number of the evaluation time windows is increased in the time axis direction.

An evaluation time window, for example, corresponds to a final determination result of an evaluation time period, and the evaluation time period is included in the evaluation time window. In the embodiment, the evaluation time window corresponds to the final determination result of the last one of the time periods. For example, the evaluation time window W1 corresponds to the final determination result of the time period t4, the evaluation time window W2 corresponds to the final determination result of the time period t5, the evaluation time window W3 corresponds to the final determination result of the time period t6, the evaluation time window W4 corresponds to the final determination result of the time period t7, the evaluation time window W5 corresponds to the final determination result of the time period t8, and so on. However, the invention is not limited thereto. In other embodiments, the evaluation time window may also correspond to the final determination result of the first one of the time periods or other one or more of the time periods. It should be noted that the time length of the evaluation time period corresponding to each evaluation time window is not necessarily the same as the time length of each time period.

For the evaluation time window W1, the processor 130 collects 5 classification results RST1 corresponding to the evaluation time window W1 (which indicate, for example, the non-occurrence of seizure, the occurrence of seizure, the occurrence of seizure, the non-occurrence of seizure and the occurrence of seizure) and then, calculates a seizure probability corresponding to the evaluation time window W1 as 3/5 according to the classification results RST1 corresponding to the evaluation time window W1.

For the evaluation time window W2, the processor 130 collects 5 classification results RST1 corresponding to the evaluation time window W2 (which indicate, for example, the occurrence of seizure, the occurrence of seizure, the non-occurrence of seizure, the occurrence of seizure and the occurrence of seizure) and then, calculates a seizure occurrence probability corresponding to the evaluation time window W2 as 4/5 according to the classification results RST1 corresponding to the evaluation time window W2.

For the evaluation time window W3, the processor 130 collects 5 classification results RST1 corresponding to the evaluation time window W3 (which indicate, for example, the occurrence of seizure, the non-occurrence of seizure, the occurrence of seizure, the occurrence of seizure, the non-occurrence of seizure), and then calculates a seizure occurrence probability corresponding to the evaluation time window W3 as 3/5 according to the classification results RST1 corresponding to the evaluation time window W3.

For the evaluation time window W4, the processor 130 collects 5 classification results RST1 corresponding to the evaluation time window W4 (which indicate, for example, non-occurrence of seizure, occurrence of seizure, occurrence of seizure, non-occurrence of seizure and occurrence of seizure), and then calculates a seizure occurrence probability corresponding to the evaluation time window W4 as 3/5 according to the classification results RST1 corresponding to the evaluation time window W4.

For the evaluation time window W5, the processor 130 collects 5 classification results RST1 corresponding to the evaluation time window W5 (which indicate, for example, the occurrence of seizure, the occurrence of seizure, the non-occurrence of seizure, the occurrence of seizure, the non-occurrence of seizure), and then calculates a seizure occurrence probability corresponding to the evaluation time window W5 as 3/5 according to the classification results RST1 corresponding to the evaluation time window W5.

After calculating the occurrence probability of the neurological event corresponding to each of the evaluation time windows W1 to W5, the processor 130 determines the final determination result corresponding to each of the evaluation time windows W1 to W5 according to the occurrence probabilities corresponding to the evaluation time windows W1 to W5.

In the embodiment, the processor 130, according to an occurrence probability corresponding to an evaluation time window, determines the final determination result corresponding to the evaluation time window. Specifically, the processor 130, according to a comparison of the occurrence probability corresponding to each of the evaluation time windows W1 to W5 with a preset threshold, determines the final determination result corresponding to each of the evaluation time windows W1 to W5. If the occurrence probability corresponding to any one of the evaluation time windows W1 to W5 is greater than the preset threshold, then the final determination result indicates that the seizure occurs. Otherwise, if the occurrence probability corresponding to any one of the evaluation time windows W1 to W5 is not greater than the preset threshold, then the final determination result indicates that the seizure does not occur.

For example, the preset threshold is set to 1/2. The occurrence probability of the neurological event corresponding to the evaluation time window W1 is 3/5, and thus, the final determination result corresponding to the evaluation time window W1 indicates the occurrence of the seizure. The occurrence probability of the neurological event corresponding to the evaluation time window W2 is 4/5, and thus, the final determination result corresponding to the evaluation time window W2 indicates the occurrence of the seizure. The occurrence probability of the neurological event corresponding to the evaluation time window W3 is 3/5, and thus, the final determination result corresponding to the evaluation time window W3 indicates the occurrence of the seizure. The occurrence probability of the neurological event corresponding to the evaluation time window W4 is 3/5, and thus, the final determination result corresponding to the evaluation time window W4 indicates the occurrence of the seizure. The occurrence probability of the neurological event corresponding to the evaluation time window W5 is 3/5, and thus, the final determination result corresponding to the evaluation time window W5 indicates the occurrence of the seizure.

In other embodiments, the final determination result corresponding to a specific evaluation time window may also be determined according to the occurrence probability corresponding to the aforementioned evaluation time window and the occurrence probability corresponding to at least one of the other evaluation time windows. Specifically, the processor 130 generates an evaluation value based on the occurrence probability corresponding to the specific evaluation time window and the occurrence probability corresponding to the at least one of other evaluation time windows through a lookup table or a conversion function, and then compares the evaluation value with a preset threshold, so as to determine the final determination result corresponding to the specific evaluation time window. However, the specific content of the lookup table or the conversion function is not limited in the invention and can be set by the persons with ordinary skills in the art based on requirements or experience.

For example, the processor 130, for instance, generates a first evaluation value based on the occurrence probability corresponding to the evaluation time window W1 and the occurrence probability corresponding to the evaluation time window W2 through a lookup table or a conversion function and then compares the first evaluation value with a preset threshold. If the first evaluation value is greater than the preset threshold, it is determined that the final determination result corresponding to the evaluation time window W1 indicates the occurrence of a neurological event (for example, the occurrence of seizure). Otherwise, if the first evaluation value is not greater than the preset threshold, it is determined that the final determination result corresponding to the evaluation time window W1 indicates that the non-occurrence of the neurological event (for example, the non-occurrence of seizure). Similarly, the processor 130, for example, generates a second evaluation value based on the occurrence probability corresponding to the evaluation time window W2 and the occurrence probability corresponding to the evaluation time window W3 through the lookup table or the conversion function, and then compares the second evaluation value with the preset threshold. If the second evaluation value is greater than the preset threshold, it is determined that the final determination result corresponding to the evaluation time window W2 indicates the occurrence of the neurological event (for example, the occurrence of seizure). Otherwise, if the second evaluation value is not greater than the preset threshold, it is determined that the final determination result corresponding to the evaluation time window W2 indicates the non-occurrence of the neurological event (for example, the non-occurrence of seizure), and so on.

In other words, as long as the final determination result corresponding to an evaluation time window is determined based on the occurrence probability of the neurological event corresponding to at least one evaluation time window and the preset threshold, the invention does not particularly limit the manner of determination.

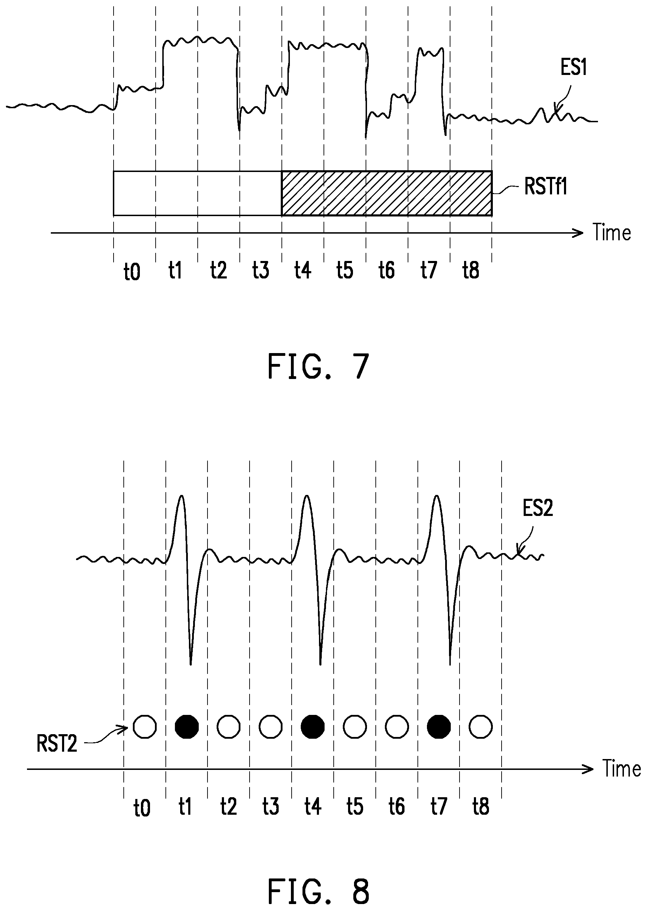

FIG. 7 is a schematic diagram illustrating displaying of final determination results according to an embodiment of the invention.

In the embodiment, the processor 130 displays the final determination results through the display 150. Referring to FIG. 7, the evaluation time windows W1 to W5 respectively correspond to the final determination results RSTf1 of the time periods t4 to t8, and the final determination results RSTf1 corresponding to the time periods t4 to t8 all indicate the occurrence of seizure. Thus, the display 150, for example, displays the EGG signal ES1 and labels a part of the final determination results RSTf1 corresponding to the time periods t4 to t8 in a specific color (which is presented by filling in with slashed lines in FIG. 7) while displaying the final determination results RSTf1, thereby indicating the occurrence of seizure during the time periods t4 to t8. Accordingly, even though the seizure occurrence appears to be intermitted during the time periods t4 to t8 (for example, the intermittence appears in the time period t6) according to the classification results RST1 of the classification model, the neurological event detection system 100 is capable of accurately determining that the occurrence during the time periods t4 to t8 is the same seizure occurrence event without intermittence.

FIG. 8 is a schematic diagram illustrating a plurality of classification results corresponding to a plurality of times according to another embodiment of the invention.

Referring to FIG. 8, each of the time periods t0 to t8 is, for example, 0.2 sec, a classification result RST2 with the occurrence of seizure is represented by a black circle, and a classification result RST2 without the occurrence of seizure is represented by a white circle. Taking an EGG signal ES2 without the occurrence of seizure, but including a plurality of spikes as an example, the processor 130 extracts a plurality of features of the EGG signal ES2 in each of the time periods t0 to t8 and classifies the features by using a classification model. As illustrated in FIG. 8, the classification results RST2 corresponding to the EGG signal ES2 from the time period t0 to the time period t8 respectively indicate the non-occurrence of seizure, the occurrence of seizure, the non-occurrence of seizure, the non-occurrence of seizure, the occurrence of seizure, the non-occurrence of seizure, the non-occurrence of seizure, the occurrence of seizure and the non-occurrence of seizure.

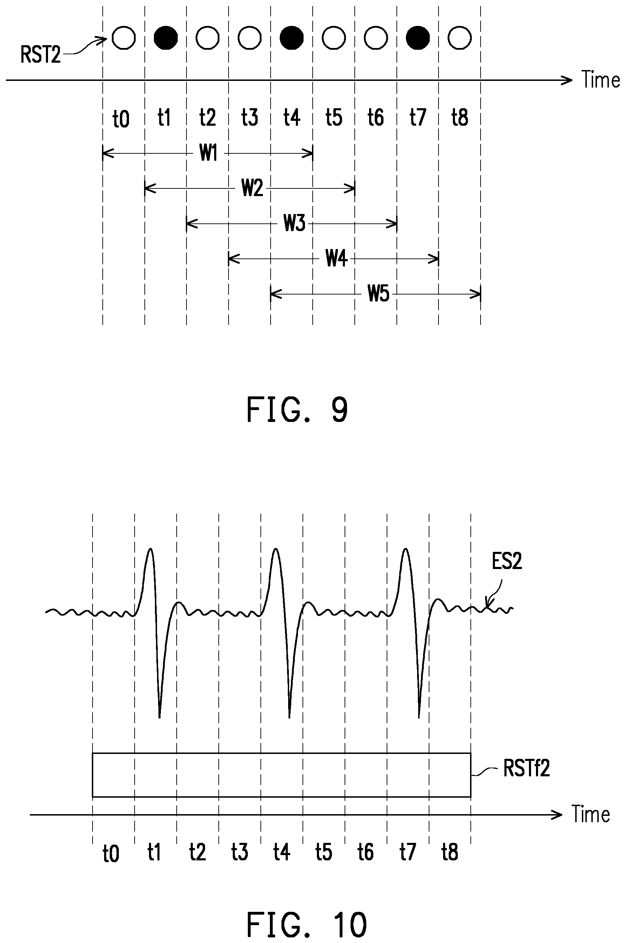

FIG. 9 is a schematic diagram illustrating the evaluation time windows according to another embodiment of the invention.

Referring to FIG. 9, the processor 130 has obtained the classification results RST2 corresponding to the EGG signal ES2 from the time period t0 to the time period t8. In the embodiment, the preset time window width is, for example, 1 sec. Thus, one evaluation time window includes 5 time periods, and each of the time periods includes a classification result. For example, the evaluation time window W1 includes the time periods t0 to t4, the evaluation time window W2 includes the time periods t1 to t5, the evaluation time window W3 includes the time periods t2 to t6, the evaluation time window W4 includes the time periods t3 to t7, the evaluation time window W5 includes the time periods t4 to t8, and so on.

For the evaluation time window W1, the processor 130 collects the 5 classification results RST2 corresponding to the evaluation time window W1 (which indicate, for example, the non-occurrence of seizure, the occurrence of seizure, the non-occurrence of seizure, the non-occurrence of seizure and the occurrence of seizure), and then calculates a seizure probability corresponding to the evaluation time window W1 as 2/5 according to the classification results RST2 corresponding to the evaluation time window W1.

For the evaluation time window W2, the processor 130 collects 5 classification results RST2 corresponding to the evaluation time window W2 (which indicate, for example, the occurrence of seizure, the non-occurrence of seizure, the non-occurrence of seizure, the occurrence of seizure and the non-occurrence of seizure), and then calculates a seizure probability corresponding to the evaluation time window W2 as 2/5 according to the classification results RST2 corresponding to the evaluation time window W2.

For the evaluation time window W3, the processor 130 collects 5 classification results RST2 corresponding to the evaluation time window W3 (which indicate, for example, the non-occurrence of seizure, the non-occurrence of seizure, the occurrence of seizure, the non-occurrence of seizure and the non-occurrence of seizure), and then calculates a seizure probability corresponding to the evaluation time window W3 as 1/5 according to the classification results RST2 corresponding to the evaluation time window W3.

For the evaluation time window W4, the processor 130 collects 5 classification results RST2 corresponding to the evaluation time window W4 (which indicate, for example, the non-occurrence of seizure, the occurrence of seizure, the non-occurrence of seizure, the non-occurrence of seizure and the occurrence of seizure) and then, calculates a seizure probability corresponding to the evaluation time window W4 as 2/5 according to the classification results RST2 corresponding to the evaluation time window W4.

For the evaluation time window W5, the processor 130 collects 5 classification results RST2 corresponding to the evaluation time window W5 (which indicate, for example, the occurrence of seizure, the non-occurrence of seizure, the non-occurrence of seizure, the occurrence of seizure and the non-occurrence of seizure), and then calculates a seizure probability corresponding to the evaluation time window W5 as 2/5 according to the classification results RST2 corresponding to the evaluation time window W5.

After calculating the occurrence probability of the neurological event corresponding to each of the plurality of evaluation time windows W1 to W5, the processor 130 determines the final determination result corresponding to each of the evaluation time windows W1 to W5 according to the occurrence probabilities corresponding to the evaluation time windows W1 to W5.

In the embodiment, the processor 130, according to an occurrence probability corresponding to an evaluation time window, determines the final determination result corresponding to the evaluation time window. Specifically, the processor 130 compares the occurrence probability corresponding to each of the evaluation time windows W1 to W5 with a preset threshold, so as to determine the final determination result corresponding to each of the evaluation time windows W1 to W5. If the occurrence probability corresponding to any one of the evaluation time windows W1 to W5 is greater than the preset threshold, the final determination result indicates that the seizure occurs. Otherwise, if the occurrence probability corresponding to any one of the evaluation time windows W1 to W5 is not greater than the preset threshold, then the final determination result indicates that the seizure does not occur.

For example, the preset threshold is set to 1/2. The occurrence probability of the neurological event corresponding to the evaluation time window W1 is 2/5, and thus, the final determination result corresponding to the evaluation time window W1 indicates the non-occurrence of seizure. The occurrence probability of the neurological event corresponding to the evaluation time window W2 is 2/5, and thus, the final determination result corresponding to the evaluation time window W2 indicates the non-occurrence of seizure. The occurrence probability of the neurological event corresponding to the evaluation time window W3 is 1/5, and thus, the final determination result corresponding to the evaluation time window W3 indicates the non-occurrence of seizure. The occurrence probability of the neurological event corresponding to the evaluation time window W4 is 2/5, and thus, the final determination result corresponding to the evaluation time window W4 indicates the non-occurrence of seizure. The occurrence probability of the neurological event corresponding to the evaluation time window W5 is 2/5, and thus, the final determination result corresponding to the evaluation time window W5 indicates the non-occurrence of seizure.

FIG. 10 is a schematic diagram illustrating displaying of final determination results according to another embodiment of the invention.

In the embodiment, the processor 130 displays the final determination results through the display 150. Referring to FIG. 10, the evaluation time windows W1 to W5 respectively correspond to the final determination results RSTf2 of the time periods t4 to t8, and the final determination results RSTf2 corresponding to the time periods t4 to t8 all indicate the non-occurrence of seizure. Thus, the display 150, for example, displays the EGG signal ES2 and labels a part of the final determination results RSTf2 corresponding to the time periods t4 to t8 in a specific color (which is presented by filling in with slashed lines in FIG. 10) while displaying the final determination results RSTf2, thereby indicating the non-occurrence of seizure during the time periods t4 to t8. Accordingly, even though the seizure appears shortly in the time periods t1, t4 and t7 according to the classification results RST2 of the classification model, the neurological event detection system 100 is capable of accurately determining that no seizure event occurs during the time periods t4 to t8.

Because the neural oscillation signals of different subjects have different characteristics when the neurological event occurs, a doctor or a user may set by himself/herself the preset time window width and the preset threshold. In a condition that the preset time window width and the preset threshold are adjustable, the final determination results indicating whether the neurological event occurs may be adapted to different neural oscillation signals, so as to be more accurate. For example, through the adjustment of the preset time window width and the preset threshold, either the intermittent onset brainwaves or transient abnormal discharge of brainwaves can be accurately detected and displayed by the neurological event detection system 100. Accordingly, in some embodiment, the neurological event detection system further includes an input device (not shown), such as a keyboard a mouse, a touch screen or a microphone, and the input device is coupled to the processor 130. The user may, for example, use the input device to generate a setting signal of at least one of the preset time window width and the preset threshold. After receiving the setting signal, the processor 130 may correspondingly adjust the at least one of the preset time window width and the preset threshold according to the setting signal.

In light of the foregoing, the method for neurological event detection and the neurological event detection system provided by the embodiments of the invention, after receiving the neural oscillation signal, can not only obtain the plurality of classification results indicating whether the neurological event occurs by using the classification model, but also determine the final determination result indicating whether the neurological event occurs by combining the plurality of classification results within the preset time window width, and then display the determined final determination result. Accordingly, the whole segment where the neurological event occurs can be accurately labeled to reduce the probability of misjudgment. In addition, in some embodiments, the user can set the preset time window width and determine the threshold value used for determining the final determination results, which can be adapted to the characteristic difference of the neural oscillation signal of each person, so as to enhance usability and accuracy.

## Claims

1. A neurological event detection system (100), comprising:
a detector (110), configured to obtain a neural oscillation signal (ES1, ES2);
a processor (130), coupled to the detector (110), and configured to:
extract a plurality of features from the neural oscillation signal (ES1, ES2);
obtain a plurality of classification results (RST1, RST2) corresponding to a plurality of time periods (t0, t1, t2, t3, t4, t5, t6, t7, t8) according to the plurality of features by using a classification model, wherein the classification model is constructed based on a plurality of training data in which whether a neurological event occurs or not is known;
collect the plurality of classification results (RST1, RST2) corresponding to each of a plurality evaluation time windows (W1, W2, W3, W4, W5) according to a preset time window width, each time window including a plurality of the time periods,
wherein the classification results of the classification model are used to indicate whether the neurological event occurs or not;
calculate an occurrence probability of the neurological event corresponding to each of the evaluation time windows (W1, W2, W3, W4, W5) according to the plurality of classification results (RST1, RST2) corresponding to each of the evaluation time windows (W1, W2, W3, W4, W5), wherein each of the occurrence probabilities corresponding to each of the evaluation time windows (W1, W2, W3, W4, W5) is calculated based on a ratio of the classification results indicating occurrence of seizure in each of the evaluation time windows (W1, W2, W3, W4, W5), and the evaluation time windows (W1, W2, W3, W4, W5) are overlapping with each other; and
determine a final determination result (RSTf1, RSTf2) corresponding to each of the evaluation time windows (W1, W2, W3, W4, W5) according to the occurrence probabilities corresponding to the evaluation time windows (W1, W2, W3, W4, W5); and
a display (150), coupled to the processor (130) and configured to display (150) the final determination results (RSTf1, RSTf2),
wherein each of the final determination results (RSTf1, RSTf2) indicates whether the neurological event occurs, wherein each of the evaluation time windows (W1, W2, W3, W4, W5) corresponds to the plurality of classification results (RST1, RST2) and one single final determination result (RSTf1, RSTf2).

2. The neurological event detection system (100) according to claim 1, wherein the processor (130) is further configured to:
determine the final determination result (RSTf1, RSTf2) corresponding to a first time window according to the occurrence probability corresponding to the first time window among the evaluation time windows (W1, W2, W3, W4, W5) and a preset threshold.

3. The neurological event detection system (100) according to claim 2, wherein the processor (130) is further configured to:
according to the occurrence probability corresponding to the first time window among the evaluation time windows (W1, W2, W3, W4, W5), at least one occurrence probability corresponding to at least one second time window among the evaluation time windows (W1, W2, W3, W4, W5) and the preset threshold, determine the final determination result (RSTf1, RSTf2) corresponding to the first time window, wherein the first time window is different from the at least one second time window.

4. The neurological event detection system (100) according to claim 3, wherein the processor (130) is further configured to:
receive a setting signal and adjusting at least one of the preset time window width and the preset threshold according to the setting signal.

5. The neurological event detection system (100) according to claim 1, wherein the classification model comprises one of or a combination of an artificial neural network (ANN) model, a support vector machine (SVM) and linear classification model, a fuzzy logic model and an auto-learn system.

6. The neurological event detection system (100) according to claim 1, wherein the plurality of features comprise one of or a combination of a temporal feature and a spatial feature.

7. The neurological event detection system (100) according to claim 1, wherein the neural oscillation signal is an electroencephalography (EEG) signal.

8. The neurological event detection system (100) according to claim 1, wherein the neurological event is an event of occurrence of seizure.

## Patentansprüche

1. Ein System (100) zur Erkennung neurologischer Ereignisse, das Folgendes aufweist:
einen Detektor (110), der so konfiguriert ist, dass er ein neuronales Oszillationssignal (ES1, ES2) ermittelt;
einen Prozessor (130), der mit dem Detektor (110) gekoppelt ist und dazu ausgelegt zum:
Extrahieren einer Vielzahl von Merkmalen aus dem neuronalen Oszillationssignal (ES1, ES2);
Ermitteln einer Vielzahl von Klassifizierungsergebnissen (RST1, RST2), die einer Vielzahl von Zeitabschnitten (t0, t1, t2, t3, t4, t5, t6, t7, t8) entsprechen, und zwar nach den vielen Merkmalen unter Verwendung eines Klassifizierungsmodells, wobei das Klassifizierungsmodell auf der Grundlage einer Vielzahl von Trainingsdaten aufgebaut ist, bei denen bekannt ist, ob ein neurologisches Ereignis auftritt oder nicht;
Erfassen der Vielzahl der Klassifizierungsergebnisse (RST1, RST2), die jeweils einem der mehrere Auswertungszeitfenster (W1, W2, W3, W4, W5) nach einer voreingestellten Zeitfensterbreite entsprechen, wobei jedes Zeitfenster mehrere der Zeitabschnitte aufweist, wobei die Klassifizierungsergebnisse des Klassifizierungsmodells verwendet werden, um anzuzeigen, ob das neurologische Ereignis auftritt oder nicht;
Berechnen einer Eintrittswahrscheinlichkeit des neurologischen Ereignisses, die jedem der Auswertungszeitfenster (W1, W2, W3, W4, W5) entspricht, nach der Vielzahl von Klassifizierungsergebnissen (RST1, RST2), die jedem der Auswertungszeitfenster (W1, W2, W3, W4, W5) entsprechen, wobei jede der Eintrittswahrscheinlichkeiten, die jedem der Auswertungszeitfenster (W1, W2, W3, W4, W5) auf der Grundlage eines Verhältnisses der Klassifizierungsergebnisse berechnet wird, die das Auftreten eines Anfalls in jedem der Auswertungszeitfenster (W1, W2, W3, W4, W5) anzeigen, und wobei sich die Auswertungszeitfenster (W1, W2, W3, W4, W5) gegenseitig überlappen; und
Bestimmen eines endgültigen Bestimmungsergebnisses (RSTf1, RSTf2), das jedem der Auswertungszeitfenster (W1, W2, W3, W4, W5) entspricht, nach den den Auswertungszeitfenstern (W1, W2, W3, W4, W5) zugeordneten Auftretswahrscheinlichkeiten; und
eine Anzeige (150), die mit dem Prozessor (130) gekoppelt und so konfiguriert ist, dass sie die endgültigen Bestimmungsergebnisse (RSTf1, RSTf2) anzeigt (150),
wobei jedes der endgültigen Bestimmungsergebnisse (RSTf1, RSTf2) angibt, ob das neurologische Ereignis auftritt, wobei jedes der Auswertungszeitfenster (W1, W2, W3, W4, W5) der Vielzahl von Klassifizierungsergebnissen (RST1, RST2) und einem einzigen endgültigen Bestimmungsergebnis (RSTf1, RSTf2) entspricht.

2. System (100) zur Erkennung neurologischer Ereignisse nach Anspruch 1, wobei der Prozessor (130) ferner so konfiguriert ist zum:
Bestimmen des einem ersten Zeitfenster entsprechende endgültige Bestimmungsergebnis (RSTf1, RSTf2) nach der dem ersten Zeitfenster entsprechenden Eintrittswahrscheinlichkeit unter den Auswertungszeitfenstern (W1, W2, W3, W4, W5) und einem voreingestellten Schwellenwert.

3. System (100) zur Erkennung neurologischer Ereignisse nach Anspruch 2, wobei der Prozessor (130) ferner so konfiguriert ist, dass er:
gemäß der Eintrittswahrscheinlichkeit, die dem ersten Zeitfenster unter den Auswertungszeitfenstern (W1, W2, W3, W4, W5) entspricht, wenigstens einer Eintrittswahrscheinlichkeit, die wenigstens einem zweiten Zeitfenster unter den Auswertungszeitfenstern (W1, W2, W3, W4, W5) entspricht, sowie dem voreingestellten Schwellenwert, das dem ersten Zeitfenster entsprechende endgültige Bestimmungsergebnis (RSTf1, RSTf2) ermittelt, wobei sich das erste Zeitfenster von dem wenigstens einen zweiten Zeitfenster unterscheidet.

4. System (100) zur Erkennung neurologischer Ereignisse nach Anspruch 3, wobei der Prozessor (130) ferner so konfiguriert ist zum:
Empfangen eines Einstellsignals und Anpassen wenigstens entweder der voreingestellten Zeitfensterbreite oder dem voreingestellten Schwellenwert nach dem Einstellsignal.

5. System (100) zur Erkennung neurologischer Ereignisse nach Anspruch 1, wobei das Klassifikationsmodell eines der folgenden Elemente oder eine Kombination daraus aufweist: ein Modell eines künstlichen neuronalen Netzes (ANN), eine Support-Vektor-Maschine (SVM) und ein lineares Klassifikationsmodell, ein Fuzzy-Logik-Modell und ein selbstlernendes System.

6. System (100) zur Erkennung neurologischer Ereignisse nach Anspruch 1, wobei die Vielzahl von Merkmalen eines der folgenden oder eine Kombination davon aufweist: ein zeitliches Merkmal und ein räumliches Merkmal.

7. System (100) zur Erkennung neurologischer Ereignisse nach Anspruch 1, wobei das neuronale Oszillationssignal ein Elektroenzephalogramm (EEG)-Signal ist.

8. System (100) zur Erkennung neurologischer Ereignisse nach Anspruch 1, wobei das neurologische Ereignis das Auftreten eines Anfalls ist.

## Revendications

1. Système de détection d'événements neurologiques (100), comprenant :
un détecteur (110), configuré pour obtenir un signal d'oscillation neuronale (ES1, ES2) ;
un processeur (130), couplé au détecteur (110), et configuré pour :
extraire une pluralité de caractéristiques du signal d'oscillation neuronale (ES1, ES2) ;
obtenir une pluralité de résultats de classification (RST1, RST2) correspondant à une pluralité de périodes de temps (t0, t1, t2, t3, t4, t5, t6, t7, t8) en fonction de ladite pluralité de caractéristiques à l'aide d'un modèle de classification, dans lequel ledit modèle de classification est construit sur la base d'une pluralité de données d'apprentissage pour lesquelles on sait si un événement neurologique se produit ou non ;
collecter la pluralité de résultats de classification (RST1, RST2) correspondant à chacune d'une pluralité de fenêtres temporelles d'évaluation (W1, W2, W3, W4, W5) selon une largeur de fenêtre temporelle prédéfinie, chaque fenêtre temporelle comprenant une pluralité des périodes de temps, les résultats de classification du modèle de classification étant utilisés pour indiquer si l'événement neurologique se produit ou non ;
calculer une probabilité d'occurrence de l'événement neurologique correspondant à chacune des fenêtres temporelles d'évaluation (W1, W2, W3, W4, W5) en fonction de la pluralité de résultats de classification (RST1, RST2) correspondant à chacune des fenêtres temporelles d'évaluation (W1, W2, W3, W4, W5), chacune des probabilités d'occurrence correspondant à chacune des fenêtres temporelles d'évaluation (W1, W2, W3, W4, W5) est calculée sur la base d'un rapport des résultats de classification indiquant la survenue d'une crise dans chacune des fenêtres temporelles d'évaluation (W1, W2, W3, W4, W5), et les fenêtres temporelles d'évaluation (W1, W2, W3, W4, W5) se chevauchent les unes avec les autres ; et
déterminer un résultat de détermination final (RSTf1, RSTf2) correspondant à chacune des fenêtres temporelles d'évaluation (W1, W2, W3, W4, W5) en fonction des probabilités d'occurrence correspondant aux fenêtres temporelles d'évaluation (W1, W2, W3, W4, W5) ; et
un écran (150), couplé au processeur (130) et configuré pour afficher (150) les résultats de détermination finaux (RSTf1, RSTf2),
chacun des résultats de détermination finaux (RSTf1, RSTf2) indiquant si l'événement neurologique se produit, chacune des fenêtres temporelles d'évaluation (W1, W2, W3, W4, W5) correspondant à la pluralité de résultats de classification (RST1, RST2) et à un seul résultat de détermination final (RSTf1, RSTf2).

2. Système de détection d'événements neurologiques (100) selon la revendication 1, dans lequel le processeur (130) est en outre configuré pour :
déterminer le résultat de détermination final (RSTf1, RSTf2) correspondant à une première fenêtre temporelle en fonction de la probabilité d'occurrence correspondant à cette première fenêtre temporelle parmi les fenêtres temporelles d'évaluation (W1, W2, W3, W4, W5) et d'un seuil prédéfini.

3. Système de détection d'événements neurologiques (100) selon la revendication 2, dans lequel le processeur (130) est en outre configuré pour :
en fonction de la probabilité d'occurrence correspondant à la première fenêtre temporelle parmi les fenêtres temporelles d'évaluation (W1, W2, W3, W4, W5), d'au moins une probabilité d'occurrence correspondant à au moins une deuxième fenêtre temporelle parmi les fenêtres temporelles d'évaluation (W1, W2, W3, W4, W5) et le seuil prédéfini, déterminer le résultat de détermination final (RSTf1, RSTf2) correspondant à la première fenêtre temporelle, la première fenêtre temporelle étant différente de la au moins une deuxième fenêtre temporelle.

4. Système de détection d'événements neurologiques (100) selon la revendication 3, dans lequel le processeur (130) est en outre configuré pour :
recevoir un signal de réglage et ajuster au moins l'une parmi la largeur de fenêtre temporelle prédéfinie et le seuil prédéfini en fonction du signal de réglage.

5. Système de détection d'événements neurologiques (100) selon la revendication 1, dans lequel le modèle de classification comprend l'un des éléments suivants ou une combinaison de ceux-ci : un modèle de réseau neuronal artificiel (ANN), une machine à vecteurs de support (SVM), un modèle de classification linéaire, un modèle de logique floue et un système d'auto-apprentissage.

6. Système de détection d'événements neurologiques (100) selon la revendication 1, dans lequel la pluralité de caractéristiques comprend l'une des caractéristiques suivantes ou une combinaison de celles-ci : une caractéristique temporelle et une caractéristique spatiale.

7. Système de détection d'événements neurologiques (100) selon la revendication 1, dans lequel le signal d'oscillation neuronale est un signal d'électroencéphalogramme (EEG).

8. Système de détection d'événements neurologiques (100) selon la revendication 1, dans lequel l'événement neurologique est un événement correspondant à la survenue d'une crise épileptique.
